# EUROPÄISCHE PATENTSCHRIFT

(11) **EP 1 643 909 B1**
(45) Veröffentlichungstag und Bekanntmachung des Hinweises auf die Patenterteilung: **24.08.2016**
(21) Anmeldenummer: 04763213.8
(22) Anmeldetag: 14.07.2004
(51) Int. Cl.: A61B 5/145, G01N 35/00, A61B 5/15, G01N 33/487

(54) **ANALYSEGERÄT FÜR KÖRPERFLÜSSIGKEITEN**
ANALYSIS APPARATUS FOR BODY FLUIDS
APPAREIL D'ANALYSE DE LIQUIDES ORGANIQUES

(30) Priorität: 16.07.2003 DE 10332488
(43) Veröffentlichungstag der Anmeldung: 12.04.2006
(73) Patentinhaber: F.Hoffmann-La Roche AG, 4070 Basel (CH); Roche Diabetes Care GmbH, 68305 Mannheim (DE)
(72) Erfinder: HOENES, Joachim, 64673 Zwingenberg (DE); LIST, Hans, 64754 Hesseneck-Kailbach (DE); KRAEMER, Uwe, 68549 Ilvesheim (DE); MILTNER, Karl, 67227 Frankenthal (DE); RASCH-MENGES, Juergen, 68723 Schwetzingen (DE); SCHMELZEISEN-REDEKER, Guenther, 64653 Lorsch (DE); ZIMMER, Volker, 67229 Laumersheim (DE); HESS, Peter, 68165 Mannheim (DE); JANSEN, Paul, 68163 Mannheim (DE)
(74) Vertreter: Pfiz, Thomas
(86) Internationale Anmeldenummer: PCT/EP2004/007785
(87) Internationale Veröffentlichungsnummer: WO 2005/006985

(56) Entgegenhaltungen:
- EP-A- 1 238 632
- WO-A-02/078533
- WO-A-02/100274
- PATENT ABSTRACTS OF JAPAN Bd. 2000, Nr. 11, 3. Januar 2001 (2001-01-03) & JP 2000 217804 A (KDK CORP), 8. August 2000 (2000-08-08)

## Beschreibung

Die Erfindung betrifft Analysegerät für Körperflüssigkeiten, insbesondere ein tragbares Blutzuckermessgerät.

Für Diabetiker sind regelmäßige Blutzuckerkontrollen unerlässlich, um Therapie, Ernährung und Lebensrhythmus auf die jeweiligen Erfordernisse einstellen zu können. Zur Selbstkontrolle sind gleichsam als Minilabore arbeitende Handgeräte am Markt, mit denen auch von Laien die erforderlichen Schritte einfach und schnell vorgenommen werden können. Dabei werden mit einer geeigneten Testchemie versehene disposible Teststreifen bereitgehalten, um nach Beaufschlagung mit Kapillarblut einen geräteinternen Nachweis beispielsweise durch eine optische Messeinheit zu ermöglichen. Um die Blutentnahme zu erleichtern, ist am Gerät eine Stechhilfe angebracht, die eine Lanzette mit einstellbarem Stechhub gegen einen angesetzten Finger ausfährt. Hierbei ist es jedoch erforderlich, dass der Benutzer ausreichend Blut aus der Stichwunde heraus auf den Teststreifen befördert, möglichst ohne das Gerät zu verunreinigen. Bei geringer Stechtiefe sind die erzielbaren Blutmengen für herkömmliche Analysesysteme häufig nicht ausreichend, während tiefere Einstiche sehr schmerzhaft sind und zu einer Vernarbung der empfindlichen Fingerkuppen führen. Eine weitere Einschränkung liegt darin, dass die Magazinierung und Verarbeitung der Teststreifen viel Bauraum beansprucht und aufwendige Antriebe erforderlich macht.

In den zum Zeitpunkt dieser Anmeldung noch unveröffentlichten EP-Anmeldungen Nr. 02026242.4 und 02028894.0 wird bereits vorgeschlagen, Testbänder in Form von Kassetten anstelle einzelner Teststreifen zu verwenden, um Körperflüssigkeit auf einem vorzugsweise über einer Spitze freigelegten Bandabschnitt zu applizieren und zu analysieren. Einzelheiten zur Blutgewinnung sowie zu bekannten Testmedien und Nachweissystemen insbesondere für Blutglucose sind dort angegeben. Die vorbekannten Systeme beschreiben allerdings nicht die Integration der Probengewinnung in ein Testbandsystem mit automatischen Messablauf.

Dokument WO 2005/006985 offenbart ein Stechgerät mit Schwenkorm und Test bond;

Ausgehend hiervon liegt der Erfindung die Aufgabe zugrunde, die im Stand der Technik aufgetretenen Nachteile zu vermeiden und ein Gerät der eingangs genannten Art dahin zu verbessern, dass bei einfacher Handhabung unter optimierter Gewinnung der Körperflüssigkeit ein weitgehend automatischer Messablauf erreichbar ist. Insbesondere soll eine Punktion zur Blutentnahme mit geringem Einstichschmerz und unter hygienischen Bedingungen ermöglicht werden.

Zur Lösung dieser Aufgabe wird die in den unabhängigen Patentansprüchen jeweils angegebene Merkmalskombination vorgeschlagen. Vorteilhafte Ausgestaltungen und Weiterbildungen der Erfindung ergeben sich aus den abhängigen Ansprüchen.

Die Erfindung ist in den aushängenden Ansprüchen definiert.

Demnach wird ein Testsystem mit folgenden Elementen vorgeschlagen:
a) einem mit einer Aufnahme (12) für den Eingriff einer Körperpartie (66), vorzugsweise einer Fingerbeere eines Probanden versehenen Gehäuse (10),
b) einer ein in die Körperpartie (66) einstechbares Stechorgan (18) aufweisenden Stecheinheit (16),
c) einer ein Testband (22) zur Applikation von aus der Körperpartie (66) austretender Körperflüssigkeit aufweisenden Testbandeinheit (20), und
d) einer Detektionseinheit (24) zur Untersuchung der auf einem Abschnitt des Testbands (22) applizierten Körperflüssigkeit,
e) ein in eine Freigabestellung und eine Wirkstellung bezüglich der Aufnahme (12) bringbares Widerlager (14) für die Körperpartie (66),
f) das Widerlager (14) mit einer Durchstechöffiung (32) für das Stechorgan (18) versehen ist, wobei das Stechorgan (18) in einem linearen Stechhub in die gegen das Widerlager (14) anliegende Körperpartie (66) einstechbar ist und das Widerlager (14) in der Wirkstellung bei anliegender Körperpartie eine Referenzposition für einen definierten Einstich schafft.

Durch Verwendung eines beweglichen Widerlagers in der Wirkstellung bei anliegender Körperpartie wird in einfacher Weise eine Referenzposition für einen definierten Einstich geschaffen. Dadurch ist es möglich, eine minimale Stechtiefe zu wählen, die für die erforderliche Flüssigkeits- bzw. Blutgewinnung noch ausreicht, während der Einstichschmerz weitmöglich reduziert wird. In der Freigabestellung des Widerlagers wird sodann ein Freiraum für einen selbständigen Flüssigkeitsaustritt geschaffen, ohne dass das Widerlager durch die Flüssigkeit kontaminiert wird oder das nachfolgende Abholen der Flüssigkeit behindert. Dadurch lässt sich eine räumliche und verfahrensmäßige Integration erzielen, die dem Benutzer ein "One-Step-Handling" ermöglicht, bei dem nur eine Aufnahme die Schnittstelle zwischen Gerät und Körper bildet. Die Testbandeinheit ermöglicht die hygienische Verarbeitung einer hohen Anzahl von Testelementen in Form von Bandabschnitten und vereinfacht auch die Herstellung und das apparative Handling. Anzumerken bleibt, dass ohne Widerlager eine Kontrolle der Stechtiefe zwar aufwändiger, aber prinzipiell möglich ist, beispielsweise durch Überwachung der Stechkraft während des Stechvorgangs.

Vorteilhafterweise ist der mit Körperflüssigkeit zu beaufschlagende Testbandabschnitt in der Freigabestellung des Widerlagers in den Bereich der Aufnahme transportierbar, so dass gezielt an der Punktionsstelle eine Aufnahme von Körperflüssigkeit erfolgen kann.

Um eine Ortswechselbewegung seitens des Benutzers zu vermeiden, ist es von besonderem Vorteil, wenn die Stecheinheit und die Testbandeinheit mittels einer Positioniervorrichtung wechselweise in eine Funktionsstellung bezüglich der Aufnahme bringbar sind.

Eine weitere Verbesserung sieht vor, dass das Widerlager durch eine in der Wirkstellung der Aufnahme zugewandte Frontfläche der Stecheinheit gebildet ist. Dadurch lässt sich auch der Gesamtweg der Stechorganbewegung minimieren.

Gemäß einer vorteilhaften Ausführung bilden die Stecheinheit und die Testbandeinheit in definierter gegenseitiger Lagebeziehung eine relativ zu dem Gehäuse bewegbare gemeinsame Baugruppe. Hierbei ist es möglich, dass die Baugruppe aus Stecheinheit und Testbandeinheit in einer Linearführung geradlinig verschiebbar ist. Hinsichtlich der Bauraumnutzung ist es günstig, wenn die genannte Baugruppe auf einer insbesondere U-förmig gekrümmten Bahnkurve bewegbar ist. Dies lässt sich vorteilhaft dadurch realisieren, dass zwischen dem Gehäuse und einer gemeinsamen Tragstruktur für die Stecheinheit und Testbandeinheit ein Koppelgetriebe angeordnet ist.

Eine weitere vorteilhafte Ausgestaltung sieht vor, dass die Stecheinheit und die Testbandeinheit über eigens zugeordnete, vorzugsweise miteinander gekoppelte Zustellmittel auf gesonderten Bahnen wechselweise in den Bereich der Aufnahme bewegbar sind. Vorteilhafterweise sind hierbei die Zustellmittel durch einen Zahnstangen- oder Drehhebelantrieb gebildet.

Günstig ist es auch, wenn das Widerlager durch eine bewegliche Bandführung für das Testband gebildet ist Auf diese Weise können die bewegten Massen gering gehalten werden, und der Raumbedarf wird weiter verringert. Vorteilhafterweise ist das Widerlager durch eine Kurvensteuerung, insbesondere eine schaltbare Nockenscheibe in Stechrichtung begrenzt bewegbar. Der Geräteaufbau lässt sich dadurch weiter optimieren, dass die Bandführung die Stecheinheit distal vorzugsweise bogenförmig zu der Aufnahme hin überspannt.

Um beengten Platzverhältnissen Rechnung zu tragen, ist es von Vorteil, wenn die Testbandeinheit einen hin und her beweglichen Umlenkkopf zur Bildung einer im Bereich der Aufnahme positionierbaren Bandschlaufe aufweist. Eine weitere Verbesserung sieht vor, dass der Umlenkkopf zu der Aufnahme hin konvex oder winklig verjüngt ist.

Für einen kompakten Aufbau und definierte Messbedingungen ist es vorteilhaft, wenn die vorzugsweise reflexionsphotometrisch arbeitende Detektionseinheit in dem Umlenkkopf angeordnet ist.

Der Einstechvorgang lässt sich dadurch optimieren, dass das Widerlager in der Wirkstellung ein Referenzmittel für die Festlegung der Höhe des Stechhubs bildet. Eine weitere Verbesserung sieht vor, dass das Widerlager und/oder das Testband mit einer Durchstechöffnung für das Stechorgan versehen ist.

Vorteilhafterweise besitzt die Stecheinheit eine vorzugsweise von der Gehäuseaußenseite her betätigbare Verstelleinrichtung zur Einstellung des Stechhubs bezüglich des Widerlagers. Vorteilhaft ist es auch, wenn die Stecheinheit einen vorzugsweise an der Gehäuseaußenseite angeordneten Auslöser zur manuellen Auslösung des Stechhubs aufweist.

Eine bevorzugte Ausführung sieht vor, dass die Testbandeinheit durch eine Bandkassette zum abschnittsweisen Transport des Testbandes vorzugsweise mittels Wickelspulen gebildet ist. Durch die Bandkassette kann die Integrität des Bandvorrats bewahrt werden, und es ist eine gerätetechnisch einfache und hygienische Verarbeitung der als Bandabschnitte ausgebildeten Testelemente möglich.

Vorteilhafterweise ist eine Mehrzahl von Lanzetten als Stechorgan in einem vorzugsweise austauschbaren Lanzettenmagazin der Stecheinheit bevorratet. Das Testband sollte mehr als 15, vorzugsweise mehr als 50 unter Bandvorschub bezüglich der Aufnahme positionierbare Testbandabschnitte für sukzessive Untersuchungen der Körperflüssigkeit aufweisen. Hierbei ist es günstig, wenn das Verhältnis zwischen bevorrateten Stechorganen und Testbandabschnitten zwischen 1:1 und 1:50 beträgt.

Eine weitere vorteilhafte Ausgestaltung sieht vor, dass die Aufnahme ein Kompressionsorgan zur Erhöhung des Innendrucks der Körperflüssigkeit in der angepressten Körperpartie aufweist, um so auch bei geringer Stechtiefe ausreichend Flüssigkeit zu gewinnen. In diesem Zusammenhang ist es von Vorteil, wenn das Kompressionsorgan durch einen vorzugsweise konischen Pressring zur Auswölbung der Körperpartie gegen das Stechorgan gebildet ist.

In einer besonders einfachen Ausführung ist es vorgesehen, dass ein im Bereich der Aufnahme gespannter Abschnitt des Testbands als Widerlager eine Referenzposition für den Stechhub bildet.

Während bevorzugt das Widerlager zwischen der Freigabe- und Wirkstellung hin und her bewegbar ist, sieht eine denkbare kinematische Umkehr vor, dass das Widerlager durch eine Verschiebe- oder Kompressionsbewegung der Aufnahme in die Freigabe- und Wirkstellung bezüglich der Aufnahme bringbar ist.

Zur Dosierungskontrolle der applizierten Körperflüssigkeit ist es vorteilhaft, wenn die Detektionseinheit mehrere auf einem Abschnitt des Testbands nebeneinander liegende Messpunkte aufweist.

Um auch unter beengten Platzverhältnissen im Gerät eine automatische Blutauftragung realisieren zu können, ist eine Bandumlenkeinheit von Vorteil, mit welcher eine Schlaufe des Testbandes in den Bereich der Aufnahme von der Bandführung weg ausziehbar ist. Zu diesem Zweck ist es günstig, wenn die Bandumlenkeinheit eine das Testband führende, hin und her bewegliche Umlenkrolle oder eine das Testband führende federelastische Umlenkzunge aufweist.

Eine baulich einfache Realisierung sieht vor, dass die Bandumlenkeinheit einen Antrieb, vorzugsweise einen Schwenkhebelantrieb für eine hin und her gehende Bewegung zum Ausziehen und Rückholen der Bandschlaufe aufweist.

Gemäß einem weiteren Erfindungsaspekt wird vorgeschlagen, dass mindestens zwei der Gerätekomponenten wechselweise in ihre jeweilige Funktionsstellung bezüglich der Aufnahme bewegbar sind, wobei die Gerätekomponenten bevorzugt auf einer gemeinsamen Tragstruktur angeordnet sind, und die Tragstruktur zur Positionierung der Gerätekomponenten in ihrer jeweiligen Funktionsstellung gegenüber dem Gehäuse bewegbar ist Auf diese Weise lassen sich auch in dem geringen zur Verfügung stehenden Bauraum eines Handgeräts komplexe Abläufe vollständig integrieren.

In diesem Zusammenhang ist es vorteilhaft, wenn
- die Tragstruktur mittels einer Positioniervorrichtung vorzugsweise auf einer bogenförmigen Bahnkurve in die jeweilige Zielposition der Gerätekomponenten bewegbar ist;
- die Tragstruktur durch eine mit Befestigungselementen für die Gerätekomponenten versehene Plattform gebildet ist;
- die Tragstruktur Klemm- , Schraub- oder Rastorgane zur vorzugsweise lösbaren Fixierung der Gerätekomponenten aufweist;
- die Tragstruktur raster- oder gitterförmig angeordnete Befestigungspunkte für die Gerätekomponenten aufweist;
- die Testmitteleinheit ein Magazin zur Verarbeitung, insbesondere Bereitstellung und Entsorgung einer Mehrzahl von Teststreifen aufweist.

Zur verfahrensmäßigen Lösung der vorstehend genannten Aufgabe werden folgende Schritte vorgeschlagen:
a) eine Körperpartie, vorzugsweise eine Fingerbeere eines Probanden wird mit einer Aufnahme eines Gerätegehäuses in Eingriff gebracht, wobei ein Widerlager durch die Körperpartie kontaktiert wird,
b) ein Stechorgan wird in einem linearen Stechhub in die gegen das Widerlager anliegende Körperpartie eingestochen,
c) anschließend wird bei zurückgezogenem Stechorgan das Widerlager in eine Freigabestellung gebracht, und
d) aus der Körperpartie austretende Körperflüssigkeit wird auf einen in den Bereich der Aufnahme bewegten Abschnitt eines Testbandes appliziert und mittels einer Detektionseinheit analysiert.

Im Folgenden wird die Erfindung anhand der in der Zeichnung in schematischer Weise dargestellten Ausführungsbeispiele näher erläutert. Es zeigen
- Fig. 1: ein tragbares Blutzuckermessgerät für Diabetiker mit einer Stech- und Testbandeinheit in geschnittener Darstellung;
- Fig. 2a bis d: verschiedene Schritte zur Blutgewinnung an einem Blutentnahmekonus des Blutzuckermessgeräts nach Fig. 1 in einer ausschnittsweisen Vergrößerung;
- Fig. 3a und b: eine Ausführungsform eines Blutzuckermessgeräts mit einer linear verschiebbaren Baugruppe aus Stech- und Bandeinheit;
- Fig. 4a bis c: eine weitere Ausführungsform mit einer verschwenkbaren Baugruppe aus Stech- und Bandeinheit;
- Fig. 5a und b: eine weitere Ausführungsform mit jeweils gesondert verfahrbarer Stecheinheit und Bandeinheit;
- Fig. 6: einen Zahnstangenantrieb zum gekoppelten wechselweisen Verfahren von Stech- und Bandeinheit;
- Fig. 7: eine Ausführungsform eines Blutzuckermessgeräts mit hin und her bewegbarer Bandführung im Schnitt;
- Fig. 8a bis c: eine zwischen einem Gerätegehäuse und einer Innenschale bewegliche Bandführung in ausschnittsweiser perspektivischer Darstellung;
- Fig. 9a und b: eine schaubildliche Darstellung des Stechvorgangs durch das Testband und der nachfolgenden optischen Erfassung; und
- Fig. 10: einen gefederten Fingerkonus in einem stark vereinfachten Schnitt;
- Fig. 11 und 12: weitere Ausführungsbeispiele mit einem Schwenkhebelmechanismus zum Ausziehen einer Bandschlaufe aus einer Kassette in zwei verschiedenen Ansichten;
- Fig. 13 a,b,c: Ausführungsbeispiele einer Plattform zur Fixierung mehrerer Gerätekomponenten;
- Fig. 14: zwei Gerätekomponenten zur Bestückung einer Plattform in Verbindung mit einem Positionierantrieb in vereinfachter schaubildlicher Darstellung; und
- Fig. 15 a,b: die Ausführungsform nach Fig. 14 in einem Gerätegehäuse in zwei verschiedenen Funktionsstellungen.

Die in der Zeichnung dargestellten Blutzuckermessgeräte bestehen im wesentlichen aus einem Gehäuse 10, einem daran angebrachten Blutentnahmekonus 12 als Aufnahme für einen Finger eines Probanden, einem mit dem Blutentnahmekonus 12 zusammenwirkenden gehäuseinternen Widerlager 14, einer Stecheinheit 16 mit Stechorgan 18 zum Einstechen in den Finger, einer Bandeinheit 20 mit einem Testband 22 zur Applikation von aus dem Fingereinstich austretendem Blut und einer Detektionseinheit 24 zur Blutuntersuchung auf dem Testband 22.

Das Widerlager 14 ist zwischen einer Freigabestellung und einer Wirkstellung bezüglich dem Blutentnahmekonus 12 bewegbar. In der Wirkstellung lässt sich durch eine Kontaktierung der einzustechenden Körperpartie eine Bezugsposition für eine definierte Einstechtiefe festlegen, während in der Freigabestellung das Blut ungehindert aus der erzeugten Hautöffnung austreten kann und ein Freiraum zur Aufnahme eines Bluttropfens auf dem Testband 22 geschaffen wird. Der Stechvorgang und die Blutauftragung erfolgen also am selben Ort im Bereich des Blutentnahmekonus 12, so dass der Benutzer keine Ortswechselbewegung vornehmen muss und der Messablauf vollautomatisch erfolgen kann.

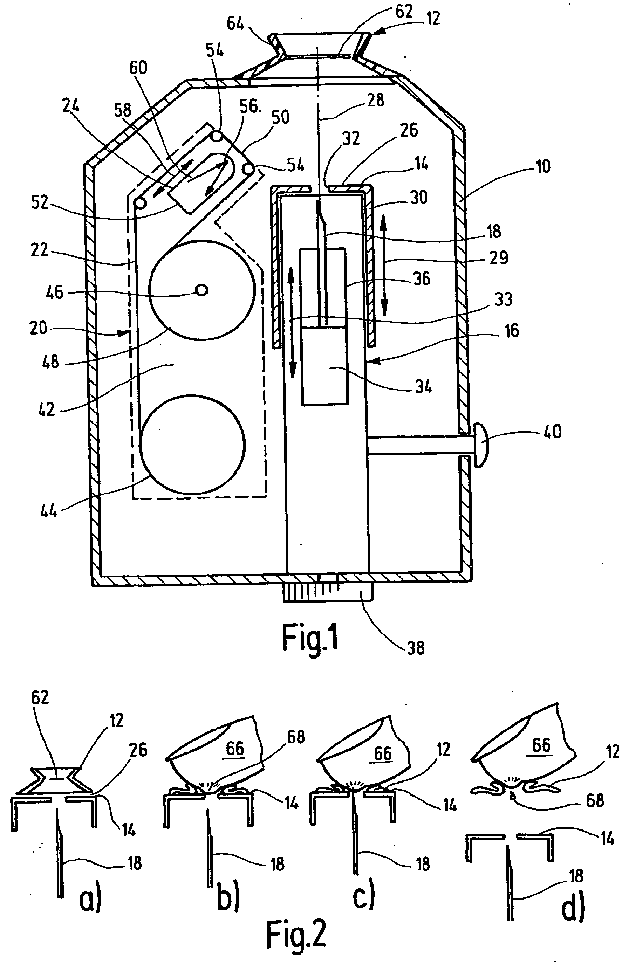

Bei der in Fig. 1 gezeigten Ausführungsform ist das Widerlager 14 durch die dem Blutentnahmekonus 12 zugewandte Stirnfläche 26 einer in der Stechachse 28 in Richtung des Doppelpfeils 29 verfahrbaren Frontkappe 30 der Stecheinheit 16 gebildet. Die Stirnfläche 26 ist mit einer Durchstechöffnung 32 versehen, durch die das Stechorgan 18 in einem linearen Stechhub (Doppelpfeil 33) durchstechbar ist. Zu diesem Zweck weist die Stecheinheit 16 einen Stechantrieb 34 auf, welcher mit dem durch eine Lanzette gebildeten Stechorgan 18 koppelbar ist. Dabei ist es vorgesehen, eine Mehrzahl von Lanzetten 18 in einem Vorrat 36 zu magazinieren. Zur Einstellung des Stechhubs bezüglich des Widerlagers 14 weist die Stecheinheit 16 eine von der Gehäuseaußenseite her betätigbare Verstelleinrichtung 38 auf. Als weiteres externes Bedienelement besitzt die Stecheinheit 16 einen Auslöser 40, der eine manuelle Auslösung des Stechhubs des Stechantriebs 34 durch den Benutzer erlaubt.

Die Bandeinheit 20 ist durch eine Kassette 43 gebildet, in welcher das Testband 22 von einer Vorratsrolle 44 abziehbar und mittels Bandvorschubantrieb 46 auf eine Aufwickelrolle 48 aufspulbar ist. Das Testband 22 weist eine Vielzahl von Bandabschnitten 50 auf, die mit Trockenchemikalien beschichtet sind, welche mit der aufgebrachten Blutflüssigkeit reagieren und dabei eine der Blutzuckerkonzentration entsprechende, optisch detektierbare Farbänderung bewirken.

Zur Bildung einer im Bereich des Blutentnahmekonus 12 positionierbaren Bandschlaufe ist ein Umlenkkopf 52 vorgesehen. Dieser ist gegenüber dem zwischen den Umlenkrollen 54 frei liegenden Bandabschnitt 50 mit einer konvexen Führungsfläche 56 in Richtung des Doppelpfeils 58 hin und her beweglich, so dass auch bei beengtem Bauraum ein Bluttropfen im Bereich des Blutentnahmekonus 12 auf dem Bandabschnitt 50 aufgenommen werden kann. Der Umlenkkopf 52 ist zugleich als Detektionseinheit 24 mit einer reflexionsphotometrischen Messeinrichtung 60 ausgestattet, um auf dem mit Blut beaufschlagten Bandabschnitt 50 eine optische Nachweismessung durchzuführen.

Der Blutentnahmekonus 12 erhöht den Innendruck in dem angepressten Körperteil. Dadurch ist es möglich, bereits mit sehr geringen Einstechtiefen und entsprechend verringertem Einstichschmerz ausreichende Mengen an Kapillarblut zu gewinnen. Zur Anpassung an unterschiedliche Fingerkonturen ist eine zu einer Eingriffsöffnung 62 doppelt konisch zulaufende Ringlippe 64 aus einem elastomeren Material vorgesehen. Diese erzeugt bei einer Druckverformung durch einen eingreifenden Finger eine ringförmige Querkompression, die zu einer Auswölbung der Fingerkuppe gegen das Widerlager 14 bzw. das Stechorgan 18 führt. Ein solcher Fingerkonus ist aus der DE-A 100 26 172 zur Bildung eines in der Fingerkuppe gestauten Blutvolumens in Verbindung mit einem Teststreifensystem an sich bekannt. Allgemein sind auch an die Anatomie anderer Körperpartien angepasste Aufnahmen anstelle der Ringlippe 64 denkbar. So ist es beispielsweise möglich, das Gehäuse 10 bzw. die Eingriffsöffnung 62 über einen größeren Starrkonus zur Anlage an den Unterarm auszubilden. Als weitere alternative Stechorte können auch Handteller oder Ohrläppchen dienen. Generell lässt sich jedoch an der Fingerbeere am einfachsten Blut gewinnen, wenngleich dort eine hohe Konzentration von Nervenenden den Einstich vergleichsweise schmerzhafter macht.

Der Vorgang der Blutgewinnung erfolgt wie in Fig. 2 veranschaulicht. Fig. 2a zeigt das Widerlager 14 in einer Wirkstellung gegenüber dem Blutentnahmekonus 12, wobei die Stirnfläche 26 der Eingriffsöffnung 62 gehäuseinnenseitig zugewandt ist. Der Benutzer drückt entsprechend Fig. 2b einen Finger 66 gegen den elastisch verformbare Blutentnahmekonus 12 an, bis die Fingerbeere 68 die Stirnfläche 26 kontaktiert. Nun wird die Lanzette 18 um einen genau eingestellten Hubweg durch die Durchstechöffnung 32 hindurch über die Stirnfläche 26 hinaus vorgeschoben und zurückgezogen (Fig. 2c). Sodann wird das Widerlager 14 zusammen mit der Lanzette 18 von dem Blutentnahmekonus 12 weg in die Freigabestellung zurückgefahren, um einen Freiraum für den Blutaustritt zu schaffen. Während der Benutzer seinen Finger 66 noch angedrückt hält, kann der ausgetretene Bluttropfen 68 durch eine Vorschubbewegung des Umlenkkopfs 52 auf den schlaufenförmig freiliegenden Bandabschnitt 50 appliziert werden. Danach kann der Benutzer seinen Finger 66 von dem Blutentnahmekonus 12 abheben. Nach Durchführung der Nachweismessung mittels der Messeinrichtung 60 wird das Ergebnis auf einer nicht dargestellten Anzeige angezeigt. Schließlich wird der verbrauchte Bandabschnitt 50 weitergespult, so dass das Gerät wieder in Messbereitschaft steht.

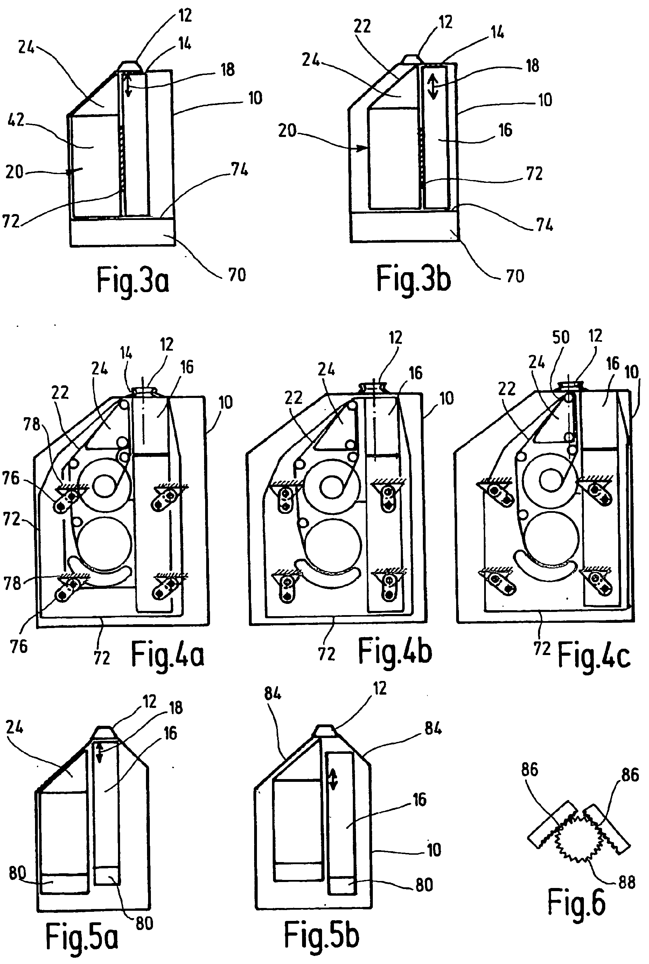

Bei den in den folgenden Figuren gezeigten Ausführungsbeispielen sind gleiche bzw. ähnliche Teile mit denselben Bezugszeichen wie vorstehend beschrieben versehen. Entsprechend Fig. 3 sind die Stecheinheit 16 und die Bandeinheit 20 (Bandkassette 42) mittels einer Positioniervorrichtung 70 wechselweise in eine Funktionsstellung relativ zu dem Blutentnahmekonus 12 bringbar. Hierfür sind die Einheiten 16, 20 über eine starre Tragstruktur 72 in fester gegenseitiger Lagebeziehung als Baugruppe in einer Linearführung 74 geradlinig verschiebbar. In der Verschiebestellung gemäß Fig. 3a kann das Stechorgan 18 betätigt werden, während in der in Fig. 3b gezeigten Stellung das Testband 22 über den spitzwinklig zulaufenden Umlenk- bzw. Messkopf 24 zur Blutapplikation aktivierbar ist.

Bei der in Fig. 4 gezeigten Ausführungsform ist die Tragstruktur 72 für die Stech- und Bandeinheit auf einer U-förmigen Bahnkurve geführt. Dabei ist die Tragstruktur 72 über vier Schwenkarme 76 mit gehäusefesten Drehgelenken 78 verbunden. In der Schwenkstellung nach Fig. 4a wird die Stecheinheit 16 gegenüber dem Blutaufnahmekonus 12 positioniert. In der mittleren Schwenkstellung gemäß Fig. 4b wird der Blutentnahmekonus 12 für den Blutaustritt freigegeben. Die Positionierung der Bandeinheit 20 erfolgt in der Schwenkstellung gemäß Fig. 4c, wobei ein schlaufenförmiger Bandabschnitt 50 in den Bereich des Blutentnahmekonus 12 eingreift.

Neben der beschriebenen linearen bzw. U-Verschiebung sind auch andere, insbesondere bogenförmige Bewegungsbahnen denkbar, um eine hinsichtlich eines kompakten Geräteaufbaus günstige Positionierung zu verwirklichen. Gemäß Fig. 5 sind zu diesem Zweck gesonderte Zustellmittel 80, 82 vorgesehen, mit denen die Stecheinheit 16 und die Bandeinheit 20 auf getrennten Bahnen wechselweise in den Bereich des Blutentnahmekonus 12 bewegbar sind. Durch die asymmetrische Gestaltung der Detektionseinheit 24 und die asymmetrische Stechposition der Stecheinheit 16 bezüglich der Zentralachse des Blutentnahmekonus 12 ist eine Minimierung der Verschiebewege möglich. Zudem lässt sich auch das Gehäuse 10 durch Wandungen 84, die gegen den Blutentnahmekonus 12 schräg zulaufen, kompakt gestalten. Um eine Kopplung der Zustellmittel 80, 82 zu erreichen, können diese entsprechend Fig. 6 durch Zahnstangen 86 gebildet sein, die über ein gemeinsames Zahnrad 88 wechselweise gegen den Blutentnahmekonus 12 verfahrbar sind.

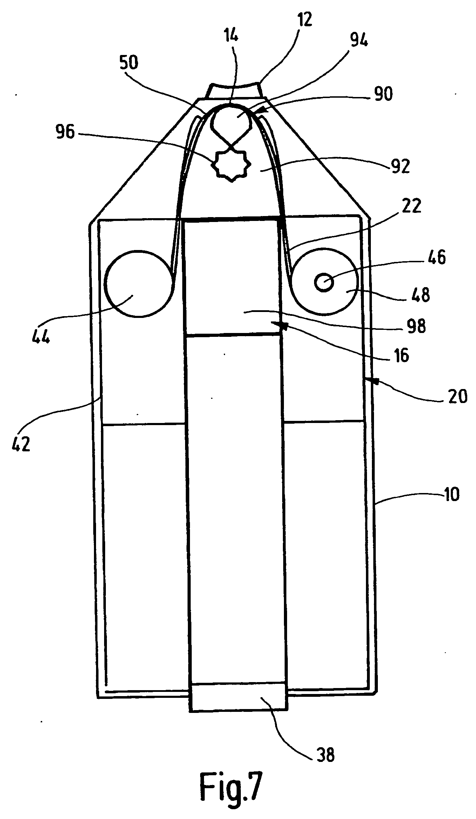

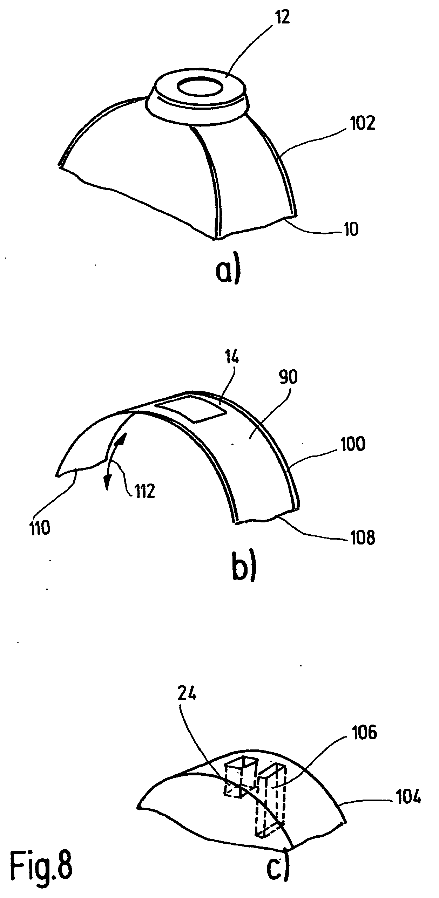

Bei der in Fig. 7 und 8 gezeigten Ausführungsform sind die Stecheinheit 16 und die Bandeinheit 20 gerätefest angeordnet, während das Widerlager 14 durch eine in Stechrichtung begrenzt bewegliche Bandführung 90 für das Testband 22 gebildet ist. Die Bandführung 90 weist ein im Kopfstück 92 der Stecheinheit 16 gelagertes Stützelement 94 auf, das unter Drehung einer Nockenscheibe 96 gegen den Blutentnahmekonus 12 hin und her verstellbar ist

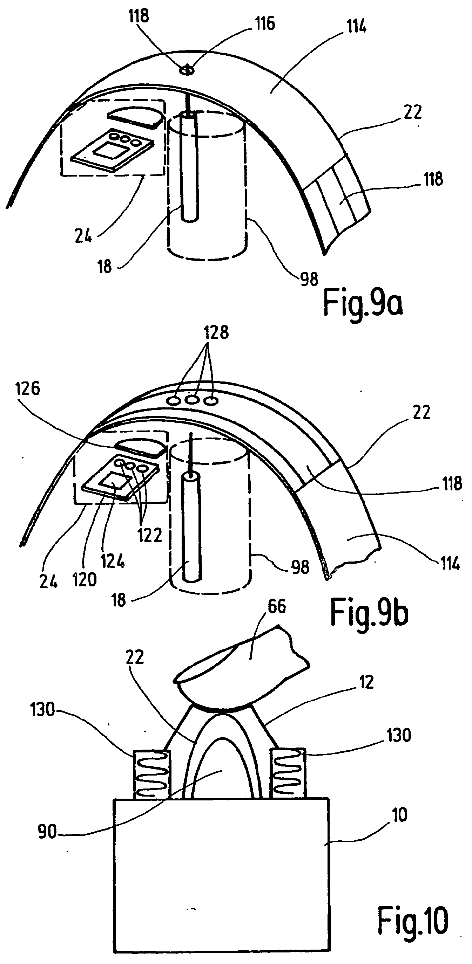

Im Ausgangszustand vor der Stechbewegung befindet sich das Stützelement 94 in der in Fig. 7 gezeigten oberen Position auf einer Nocke der Nockenscheibe 96. Der Benutzer drückt den Blutentnahmekonus 12 etwas zusammen, bis sein Finger das Widerlager 14 berührt. Nach der Stechauslösung wird eine der in dem Trommelmagazin 98 bereitgehaltenen Lanzetten durch die Bandschlaufe 50 hindurch hin und her gefahren, wie es anhand von Fig. 9 nachstehend näher erläutert ist. Um den Blutaustritt nicht zu behindern, wird anschließend das Stützelement 94 etwas abgesenkt, indem die Nockenscheibe 96 in eine Zwischenstellung zwischen den Nocken weitergedreht wird. Während einer vorgegebenen Wartezeit kann ein zu beaufschlagender Bandabschnitt 50 über den Vorschubantrieb 46 auf dem Stützelement 94 positioniert und in Anlage gehalten werden. Sodann wird die Nockenscheibe 96 weitergedreht, bis das Stützelement 94 wieder auf einer Nocke aufliegt und der angehobene Bandabschnitt 50 den Bluttropfen am Finger abholt.

Als Bandführung 90 kann auch ein schalenförmiger Führungsrahmen 100 dienen (Fig. 8b), der zwischen einer äußeren Gehäuseschale 102 (Fig. 8a) und einer inneren Geräteschale 104 (Fig. 8c) in Stechrichtung begrenzt beweglich angeordnet ist. Hierbei trägt die äußere Schale 102 den Blutentnahmekonus 12, und an die innere Schale 104 ist ein Führungsschacht 106 für die Lanzetten und eine optische Detektionseinheit 24 angekoppelt. In dieser Anordnung ist es möglich, dass der bogenförmig zu dem Blutentnahmekonus 12 hin gewölbte Führungsrahmen 100 nur an einem Ende 110 um einen gegebenen Schwenkhub 112 beweglich ist, während das andere Rahmenende 108 feststehend an der Bandkassette 42 angeordnet ist.

Wie in Fig. 9a gezeigt, kann der Stechvorgang direkt durch eine dünne Trägerfolie 114 des Testbands 22 hindurch erfolgen, wobei zweckmäßig ein in der Stechachse positioniertes Pilotloch 116 eine ungewollte Beschädigung der Lanzettenspitze 118 vermeidet. Die Trägerfolie 114 ist abschnittsweise mit einem Testfeld 118 beschichtet, das nach dem Stechvorgang unter Bandvorschub über geeignete Steuermittel wie beispielsweise eine Lichtschranke oder mechanische Lochmaskenführung in die in Fig. 9b gezeigte Blutaufnahme- und Messposition weitertransportiert wird. Die für einen berührungslosen Nachweis eingesetzte optische Detektionseinheit 24 umfasst eine Messplatine 120 mit drei Lichtquellen (LEDs 122) und einem Photosensor 124 sowie einer Sammeloptik (Linse 126). Die LEDs 122 sind in Reihe angeordnet und erzeugen entsprechend über die Linse 126 drei in Bandlaufrichtung gesehen hintereinander liegende Leuchtflecke 128. Diese befinden sich im Bereich der Stechachse auf dem Testfeld 118 über der transparenten Trägerfolie 114. Um eine eindeutige Zuordnung für das von den Leuchtflecken 128 remittierte Licht mit nur einem Sensor 124 erreichen zu können, werden die LEDs 122 nacheinander angesteuert. In dieser Anordnung dient die mittlere LED zur eigentlichen Nachweismessung, während die beiden äußeren LEDs eine Dosierungskontrolle ermöglichen. Dabei wird eine Fehldosierung erkannt, wenn eine unsymmetrische Signalverteilung vorliegt. Das Testfeld 118 enthält Trockenchemikalien, die auf den Analyten (Glucose) in der applizierten Blutflüssigkeit ansprechen und zu einer Veränderung der Lichtrückstrahlung führen.

In der in Fig. 10 stark vereinfachten Darstellung ist ein Blutaufnahmekonus 12 gezeigt, der über federnde Elemente 130 gegenüber dem Gehäuse 10 so abgestützt ist, dass eine Relativbewegung gegenüber dem Testband 22 bzw. dessen Führung 90 unter dem Druck des Fingers 66 möglich ist. Die Federelemente 130 können ähnlich einem Faltenbalg ausgebildet sein und sollten mit zunehmender Verformung nichtlinear härter werden. Dabei ist es auch denkbar, dass das Widerlager 14 durch die gespannte Trägerfolie 114 gebildet ist und durch eine Hin- und Herbewegung des Blutaufnahmekonus 12 in die Wirk- bzw. Freigabestellung bezüglich der Aufnahme bringbar ist.

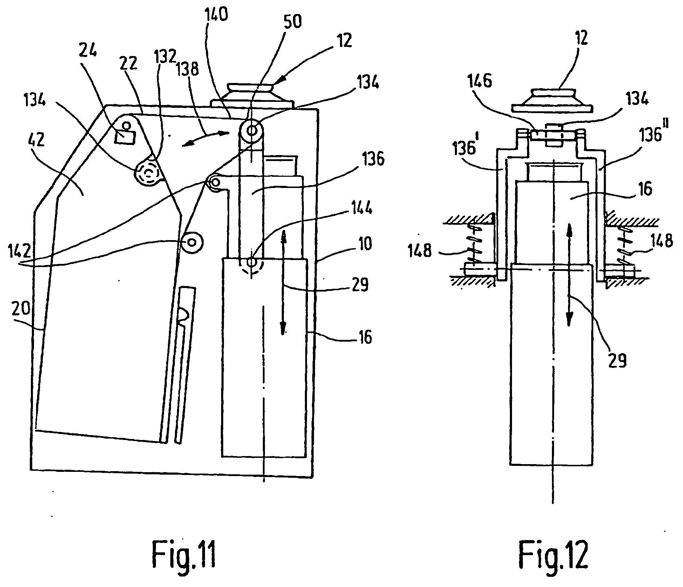

Bei dem in Fig. 11 und 12 gezeigten Ausführungsbeispiel besitzt die Bandkassette 42 an geeigneter Stelle eine Freisparung 132, in die beim Einlegen der Kassette eine Umlenkrolle 134 eingreift. Diese Rolle 134 ist am freien Ende eines Schwenkhebels 136 gelagert, der in Richtung des Pfeils 138 eine hin und her gehende Schwenkbewegung ausführen kann. Wird nun nach dem Einstich in die Haut die Stecheinheit 16 oder zumindest der vordere Teil davon zurück bewegt (Pfeil 29), so kann durch Ausschwenken der Rolle 134 eine Bandschlaufe 140 aus der Kassette 42 gezogen und an den Ort der Blutgewinnung gebracht werden. Der Hub, um den die Stecheinrichtung bewegt werden muss, wird im Wesentlichen durch den Durchmesser der Rolle 134 definiert, welcher vergleichsweise gering sein kann (z.B. 3 mm). Damit das Testband 22 während dieser Bewegung keine ungünstigen Kräfte z.B. auf eine Dichtung der Kassette 42 ausübt und andererseits nicht an sonstigen Gerätestrukturen 16 schleift, sind zwei weitere Führungsrollen 142 vorgesehen.

Beim Ausschwenken zieht der Hebel 136 mit der Rolle 134 frisches Band 22 von der Vorratsrolle, die nicht angetrieben, sondern nur gebremst ist. Dabei wird sichergestellt, dass nach der Schwenkbewegung ein mit Nachweischemikalien versehenes Testfeld 50 unter dem Konus 12 liegt. Auf dem Schwenkweg wird die Rolle 134 gezielt in einem Bogen von unten an die gestochene Haut herangeführt, so dass sie (von der Kassette 42 aus gesehem) jenseits des Einstichortes am Finger ankommt. Dadurch die Bandschlaufe 140 nicht nur gleichsam auf der Haut ausgerollt, sondern sanft von unten an die Blutaustrittsstelle herangeführt. Dieses geschieht mit Motorkraft eines Schwenkantriebs 144.

Mit Erreichen dieser Position wird der Hebel 136 vom motorischen Antrieb abgekoppelt, wobei er weiterhin unter schwacher Federspannung nach außen steht. Damit wird das Band 33 unter Zugspannung gehalten. Einen solchen Antrieb, der sich selber auskoppelt kann man leicht mit einem Nocken erreichen.

Danach wird das Band 22 von der Aufwickelrolle eingespult, und zwar so weit, dass die mit Blut benetzte Stelle vor der Detektionseinheit 24 zu liegen kommt. Bei geeigneter Anordnung von Konus 12, Kassette 42 und Drehpunkt des Hebels 136 schmiegt sich das Band 22 in diesem Moment genau über die Kassettenspitze, die es präzise vor der Optik der Detektionseinheit 24 positioniert. Während dieser Ablaufphase wird die Rolle 134 auf ihrem nun nur federbelasteten Hebel 136 wie von einem Flaschenzug eingeholt Die Federbelastung sorgt dabei dafür, dass das Band niemals lose fällt. Ein besonderer Vorteil liegt darin, dass auch in solchen Fällen, in denen die zu stechende Körperpartie nicht weit in das Gerät hinein exponiert werden kann, eine saubere Bandbewegung erfolgt, ohne Gerätestrukturen mit Blut zu kontaminieren.

Eine bevorzugte Ausführungsform gemäß Fig. 12 besteht darin, dass die Rolle 134 in der Aussparung 132 der Kassette 42 eingeklipst ist. Beim Einlegen der Kassette 42 wird sie auf den Lagerzapfen 146 am Hebelarm 136' selbsttätig aufgesteckt, ohne dass sich der Anwender darum kümmern müsste. Am Ende des Bewegungszyklus wird die Rolle wiederum motorisch in die Verrastung eingeklipst, so dass sie bei Entnahme der Kassette 42 aus dem Gehäuse 10 als Bestandteil der Kassette hygienisch entsorgt werden kann.

Damit sich die Rolle 134 nicht durch Schwerkraft oder Stöße vom Zapfen 146 lösen kann, muss sie axial geführt werden. Dies lässt sich dadurch realisieren, dass entlang der Bewegungsbahn der Rolle 134 nicht gezeigte Führungsrippen verlaufen, die die Bewegungsfreiheit in axialer Richtung beschränken - und dabei gegebenenfalls zugleich eine Hilfsführung für das Band bieten. Oder aber der Hebel 136 ist zweigeteilt (136' und 136"). Der eine Hebelarm 136' mit dem Lagerzapfen 146 ist dabei schwenkbar angetrieben, während der zweite Hebelarm 136" unter leichter Federlast zur Kassette hin gedrängt wird, dort aber an einem festen Anschlag gestoppt wird. Schwenkt nun der erste Hebel 136' mit der Rolle 134 von der Kassette 42 weg, so nimmt er mit dem überstehenden Ende des Lagerzapfens 146 den zweiten Hebel 136" mit, der so einen axialen Anschlag für die Rolle 134 liefert.

Die beiden Hebel 136', 136" sind beidseitig der Stecheinheit 16 drehbar gelagert, wobei die Lagerungen miteinander fluchten. Eine besondere Ausführungsform erlaubt eine Bewegung der Lagerachsen hin zum Fingerkonus 12 gegen die Rückstellkraft der Federn 148. Wenn der Doppelhebel das Band in die Abholposition ausgeschwenkt hat, steht die Rolle 134 genau unter dem Konus 12, berührt aber die Fingerbeere noch nicht. Das Testfeld auf dem Testband 22 umschlingt zumindest die obere Hemisphäre der Rolle 134. Ist dieser Zustand erreicht, bewegt sich die Stecheinheit 16 nach oben und stößt dabei an dem gefederten Doppelhebel 136 an, worauf dieser mitgenommen wird. Dadurch tupft die Rolle 134 mit dem umschlingenden Testfeld in den ausgetretenen Blutstropfen. Anschließend bewegt sich die Stecheinheit 16 zurück, wobei die Federn 148 den Hebel 136 und damit die Rolle 134 wieder vom Finger wegholen.

Eine weitere nicht gezeigte Ausführungsform verwendet anstelle einer Rolle 134 eine einfache Blechzunge, die das Band 22 in ihrer (steifen) Längsrichtung ausfährt, aber in ihrer Querrichtung, in der sie biegeweich ist, an die Haut anfedert. Denkbar ist es auch, dass die Kassette 42, die Detektionseinheit 24 und die Stecheinheit 136 nicht jn einer Ebene liegen. Die Flexibilität des Bandes 22 erlaubt es dann, unter seitlicher Auslenkung quer zur Bandschlaufe eine Einstichstelle im Winkel zur Kassettenebene anzufahren.

Für den Fall einer sehr schnellen chemischen Reaktion auf dem Testelement kann es nötig sein, bereits den Blutauftrag mit der Messeinrichtung zu verfolgen. Dafür kann eine Bandschlaufe wie vorstehend zu Fig. 1 beschrieben durch eine als Umlenkkopf 56 ausgebildete Detektionseinheit zum Blutaufnahmeort transportiert werden. Dabei ist es vorteilhaft, wenn die Bandschlaufe auf dem Umlenkkopf festgeklemmt und in diesem fixierten Zustand zur Probe gebracht wird. Diese Festklemmung kann durch eine Gabel erreicht werden, die im Verfahrweg des Umlenkkopfes angeordnet ist und durch diesen gegen eine Federlast mit zur Blutauftragsstelle mitgenommen wird. Der Umlenkkopf greift dabei zwischen den Gabelschenkeln ein, so dass das Testband fest und unverrückbar eingeklemmt wird.

Das Herausziehen einer Bandschlaufe lässt sich auch dann vorteilhaft anwenden, wenn nicht nur das Testband zusammen mit der Detektionseinheit zum Blutauftragsort transportiert wird, sondern auch, wenn zusätzlich die Stecheinheit in enger Nachbarschaft mitbewegt wird. In diesem Fall erfolgt der Einstich durch einen zwischen Testfeldern liegenden Trägerbandabschnitt hindurch. Nach dem Rückzug zur Freigabe des Blutaustrittes wird das nächste Testfeld hinter die Wunde gespult, das Blut wird durch Vorfahren aufgenommen und nach erneutem Rückzug das Testfeld über die Detektionseinheit gespult. Auch hierbei ist neben dem bloßen Umspulen des Bandes von der Vorrats- auf die Aufwickelrolle eine gesonderte Querbewegung eines Bandabschnittes in Form einer Bandschlaufe hilfreich.

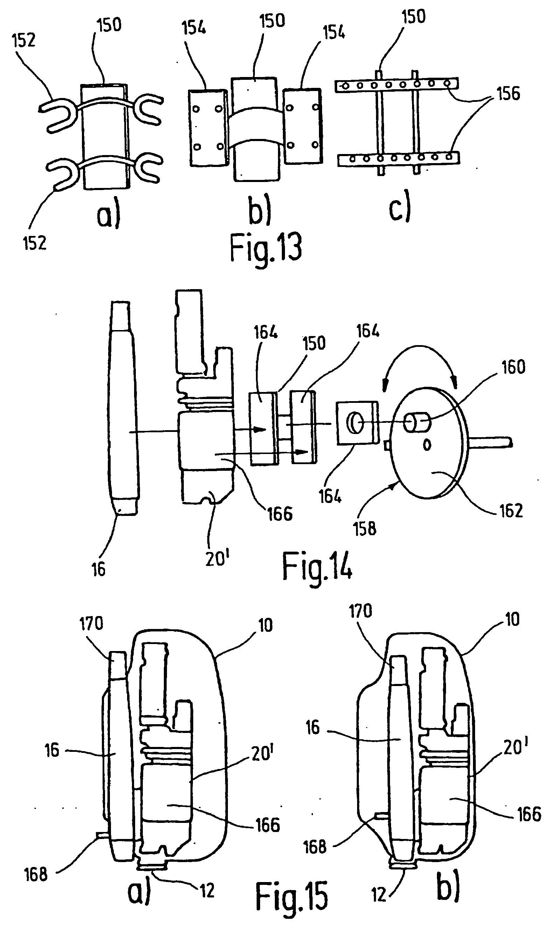

Bei dem in Fig. 13 bis 15 gezeigten Ausführungsbeispiel ist als "Kernstück" eine bewegliche Plattform 150 vorgesehen, an welcher die benötigten Gerätekomponenten zur Durchführung der Blutgewinnung und Analyse oder sonstiger Gerätefunktionen befestigt werden können. Als "Plattform" kann jedwede mögliche Ausführung einer Tragstruktur für daran zu befestigende Komponenten in Frage kommen. In Fig. 13a ist beispielsweise eine Plattform 150 mit daran angebrachten Klemmvorrichtungen 152 veranschaulicht, während Fig. 13b eine Basis 150 mit daran angebrachten Montageplatten 154 und Fig. 13c eine Basis 150 in Form einer Gitterkonstruktion mit variablen Befestigungspunkten 156 zeigen.

Die Plattform 150 ist in dem Gehäuse 10 beweglich, um die daran befestigten Komponenten zur Blutgewinnung, Blutabholung oder sonstigen Funktionen zu positionieren. Die Zielposition ist insbesondere bezüglich der Fingeraufnahme 12 des Gehäuses 10 definiert. Dabei ist die Bewegung der Plattform 150 möglichst so durchzuführen, dass die daran befestigten Komponenten in distaler Richtung an die entsprechenden Zielpositionen angefahren werden. Vorzugsweise wird hierfür eine bogenförmige (Halbkreis-)Bewegung gewählt. Bei mehr als zwei Komponenten kann die bogenförmige Bewegung entsprechend oft hintereinander ausgeführt werden. Hierbei kann die Bogenweite der einzelnen Bewegungen auch an die erforderlichen Dimensionen/Abmessungen der verwendeten Komponenten angepasst sein, d.h. es werden unterschiedlich weite Bögen gefahren.

Die bewegliche Plattform 150 ist entweder als Einheit oder in den einzelnen Befestigungspunkten der angebrachten Komponenten verstell- bzw. justierbar. Die Bewegungsrichtung der Verstellbewegung verläuft vorzugsweise axial zur Aufnahme 12, so dass eine "Höhenjustage" der gesamten oder der einzelnen Komponenten möglich ist. Die Justage erfolgt entweder manuell, z.B. während der Montage, oder auch automatisch während des Messbetriebs. Denkbar ist auch eine individuelle Anpassung an den jeweiligen Anwender.

Wie in Fig. 14 veranschaulicht, kann eine einfache elektromechanische Positioniervorrichtung 158 die Plattform 150 zur Positionierung der darauf befestigten Stecheinheit und Testmitteleinheit 20'. Die Bewegung erfolgt hier durch Drehung eines Zapfens 160 auf einer Scheibe 162 in einer Kulisse 164. Möglich ist es beispielsweise auch, die Bewegung durch die Umsetzung der Drehbewegung eines Hebels, oder direkt durch die Drehung eines Zahnrades in einer Kulisse auf die bewegliche Plattform 150 zu übertragen (nicht gezeigt). Wichtig ist hierbei, dass die jeweiligen Endpositionen stabil sind.

An der beweglichen Plattform 150 sind Fixierflächen 164 für die zu bewegenden Komponenten 16, 20' vorgesehen. Es versteht sich, dass jedwede geeignete Befestigungsorgane entsprechend den Erfordernissen der jeweiligen Gerätekomponenten eingesetzt werden können und daher hier nicht näher beschrieben werden. Speziell gezeigt ist die modulare Plattform-Kombination von Einzelkomponenten 16, 20', welche als solche unter der Marke Accu-Chek® Compact am Markt bereits bekannt sind. Die Stechhilfe 16 ermöglicht das Auslösen einer Stechbewegung einer Lanzette, während das Modul 20' ein Trommelmagazin 166 mit Abtrieb und Schubstange für die automatische Blutaufnahme auf einzelnen Teststreifen sowie eine Messoptik als Detektionseinheit zur Blutglukoseauswertung umfasst.

Fig. 15 zeigt das zusammengesetzte Gerät. Die Stechhilfe 16 ragt im gezeigten Beispiel endseitig aus dem Gehäuse heraus (Fig. 15a). So ist der Knopf 168 zum Spannen der Stechhilfe weiterhin vom Anwender bedienbar und der Lanzettenwechsel kann bei Bedarf wie gewohnt durch das Abnehmen der unteren Kappe 170 geschehen. Nach dem Start der Messung dreht sich das hinter der beweglichen Plattform 150 befindliche Rad 162 und verschiebt über den Zapfen 160 und eine die Kulisse 164 die Stechhilfe 16 bogenförmig in die Stechposition über der Aufnahme 12 (Fig. 15b). Sodann kann der Stechvorgang nun ausgelöst werden, entweder manuell durch den Anwender oder durch eine automatisierte Funktion.

Nachdem der Stechvorgang abgeschlossen ist, kann sich die bewegliche Plattform 150 mit den daran befestigten Komponenten 16, 20' durch entgegengesetzte Drehung der Positioniervorrichtung 158 in die Ausgangsposition zurück bewegen. Die Komponente 20' mit der Trommel 166 befindet sich nun über der Aufnahme 12. In dieser Stellung kann ein Teststreifen aus der Trommel 166 heraus geschoben werden, welcher das Probenvolumen über eine Kapillare aufnimmt und zu einem Testfeld befördert. Die unterhalb der Trommel gelegene Messoptik kann dann die Messung durchführen. Anschließend wird der gebrauchte Teststreifen zur Entsorgung wieder in die Trommel zurückgezogen.

Auf diese Weise ist eine vollständige Integration der folgenden Arbeitsschritte in einem einzigen Gerät möglich:
- automatische Blutgewinnung
- automatischer Blutauftrag auf einen Teststräger
- Bevorratung von unbenutzten Testträgern
- Entsorgung von gebrauchten Testträgern.
Für den Anwender vereinfacht sich das Handling erheblich, und der gesamte Messablauf kann weitgehend automatisiert erfolgen.

Die Ausführungsbeispiele, die nicht unter den Schutzumfang der beigefügten Ansprüche follen, dienen lediglich zu illustrativen Zwecken und sind nicht Gegenstand der gegenwirtigen Erfindung.

Die Erfindung wird durch die folgenden Ansprüche definiert :

## Patentansprüche

1. Analysegerät für Körperflüssigkeiten, insbesondere tragbares Blutzuckermessgerät, mit
a) einem mit einer Aufnahme (12) für den Eingriff einer Körperpartie (66), vorzugsweise einer Fingerbeere eines Probanden versehenen Gehäuse (10),
b) einer ein in die Körperpartie (66) einstechbares Stechorgan (18) aufweisenden Stecheinheit (16),
c) einer ein Testband (22) zur Applikation von aus der Körperpartie (66) austretender Körperflüssigkeit aufweisenden Testbandeinheit (20), und
d) einer Detektionseinheit (24) zur Untersuchung der auf einem Abschnitt des Testbands (22) applizierten Körperflüssigkeit,
e) ein in eine Freigabestellung und eine Wirkstellung bezüglich der Aufnahme (12) bringbares Widerlager (14) für die Körperpartie (66),
**dadurch gekennzeichnet, dass**
f) das Widerlager (14) mit einer Durchstechöffnung (32) für das Stechorgan (18) versehen ist, wobei das Stechorgan (18) in einem linearen Stechhub in die gegen das Widerlager (14) anliegende Körperpartie (66) einstechbar ist und das Widerlager (14) in der Wirkstellung bei anliegender Körperpartie eine Referenzposition für einen definierten Einstich schafft.

2. Analysegerät nach Anspruch 1, **dadurch gekennzeichnet, dass** der mit Körperflüssigkeit zu beaufschlagende Testbandabschnitt in der Freigabestellung des Widerlagers (14) in den Bereich der Aufnahme (12) transportierbar ist.

3. Analysegerät nach Anspruch 1 oder 2, **dadurch gekennzeichnet, dass** die Stecheinheit (16) und die Testbandeinheit (20) mittels einer Positioniervorrichtung (70) wechselweise in eine Funktionsstellung bezüglich der Aufnahme (12) bringbar sind.

4. Analysegerät nach einem der Ansprüche 1 bis 3, **dadurch gekennzeichnet, dass** das Widerlager (14) durch eine in der Wirkstellung der Aufnahme (12) zugewandte Frontfläche (26) der Stecheinheit (16) gebildet ist.

5. Analysegerät nach einem der Ansprüche 1 bis 4, **dadurch gekennzeichnet, dass** die Stecheinheit (16) und die Testbandeinheit (20) in definierter gegenseitiger Lagebeziehung eine relativ zu dem Gehäuse (10) bewegbare gemeinsame Baugruppe bilden.

6. Analysegerät nach einem der Ansprüche 1 bis 5, **dadurch gekennzeichnet, dass** die Baugruppe aus Stecheinheit (16) und Testbandeinheit (20) in einer Linearführung (74) geradlinig verschiebbar ist.

7. Analysegerät nach einem der Ansprüche 1 bis 5, **dadurch gekennzeichnet, dass** die die Baugruppe aus Stecheinheit (16) und Testbandeinheit (20) auf einer insbesondere U-förmig gekrümmten Bahnkurve bewegbar ist.

8. Analysegerät nach einem der Ansprüche 1 bis 7, **dadurch gekennzeichnet, dass** zwischen dem Gehäuse (10) und einer gemeinsamen Tragstruktur (72) für die Stecheinheit (16) und Testbandeinheit (20) ein Koppelgetriebe (76,78) angeordnet ist.

9. Analysegerät nach einem der Ansprüche 1 bis 4, **dadurch gekennzeichnet, dass** die Stecheinheit (16) und die Testbandeinheit (20) Ober eigens zugeordnete, vorzugsweise miteinander gekoppelte Zustellmittel (80) auf gesonderten Bahnen wechselweise in den Bereich der Aufnahme (12) bewegbar sind.

10. Analysegerät nach Anspruch 9, **dadurch gekennzeichnet, dass** die Zustellmittel (80) durch einen Zahnstangenantrieb (86,88) oder Drehhebelantrieb gebildet sind.

11. Analysegerät nach einem der Ansprüche 1 bis 10, **dadurch gekennzeichnet, dass** das Widerlager (14) durch eine bewegliche Bandführung (90) für das Testband (22) gebildet ist.

12. Analysegerät nach einem der Ansprüche 1 bis 11, **dadurch gekennzeichnet, dass** das Widerlager (14) durch eine Kurvensteuerung (96), insbesondere eine schaltbare Nockenscheibe in Stechrichtung begrenzt bewegbar ist.

13. Analysegerät nach Anspruch 11 oder 12, **dadurch gekennzeichnet, dass** die Stecheinheit (16) und die Testbandeinheit (20) gerätefest angeordnet sind.

14. Analysegerät nach einem der Ansprüche 11 bis 13, **dadurch gekennzeichnet, dass** die Bandführung (90) die Stecheinheit (16) distal vorzugsweise bogenförmig zu der Aufnahme (12) hin überspannt.

15. Analysegerät nach einem der Ansprüche 1 bis 14, **dadurch gekennzeichnet, dass** die Testbandeinheit (20) einen hin und her beweglichen Umlenkkopf (56) zur Bildung einer im Bereich der Aufnahme (12) positionierbaren Bandschlaufe aufweist.

16. Analysegerät nach Anspruch 15, **dadurch gekennzeichnet, dass** der Umlenkkopf (56) zu der Aufnahme (12) hin konvex oder winklig verjüngt ist.

17. Analysegerät nach Anspruch 15 oder 16, **dadurch gekennzeichnet, dass** die vorzugsweise reflexionsphotometrisch arbeitende Detektionseinheit (24) in dem Umlenkkopf (56) angeordnet ist.

18. Analysegerät nach einem der Ansprüche 1 bis 17, **dadurch gekennzeichnet, dass** das Widerlager (14) in der Wirkstellung ein Referenzmittel für die Festlegung der Höhe des Stechhubs bildet.

19. Analysegerät nach einem der Ansprüche 1 bis 18, **dadurch gekennzeichnet, dass** das Testband (22) mit einer Durchstechöffnung (32) für das Stechorgan (18) versehen ist.

20. Analysegerät nach einem der Ansprüche 1 bis 19, **dadurch gekennzeichnet, dass** die Stecheinheit (16) eine vorzugsweise von der Gehäuseaußenseite her betätigbare Verstelleinrichtung (38) zur Einstellung des Stechhubs bezüglich des Widerlagers (14) aufweist.

21. Analysegerät nach einem der Ansprüche 1 bis 20, **dadurch gekennzeichnet, dass** die Stecheinheit (16) einen vorzugsweise an der Gehäuseaußenseite angeordneten Auslöser (40) zur manuellen Auslösung des Stechhubs aufweist.

22. Analysegerät nach einem der Ansprüche 1 bis 21, **dadurch gekennzeichnet, dass** die Testbandeinheit (20) durch eine Bandkassette (42) zum abschnittsweisen Transport des Testbandes vorzugsweise mittels Wickelspulen (44,48) gebildet ist.

23. Analysegerät nach einem der Ansprüche 1 bis 22, **dadurch gekennzeichnet, dass** eine Mehrzahl von Lanzetten als Stechorgan (18) in einem vorzugsweise austauschbaren Lanzettenmagazin (36) der Stecheinheit (16) bevorratet sind.

24. Analysegerät nach einem der Ansprüche 1 bis 23, **dadurch gekennzeichnet, dass** das Testband (22) mehr als 15, vorzugsweise mehr als 50 unter Bandvorschub bezüglich der Aufnahme (12) positionierbare Testbandabschnitte (50) für sukzessive Untersuchungen der Körperflüssigkeit aufweist.

25. Analysegerät nach Anspruch 24, **dadurch gekennzeichnet, dass** das Verhältnis zwischen bevorrateten Stechorganen und Testbandabschnitten zwischen 1:1 und 1:50 beträgt.

26. Analysegerät nach einem der Ansprüche 1 bis 25, **dadurch gekennzeichnet, dass** die Aufnahme (12) ein Kompressionsorgan (64) zur Erhöhung des Innendrucks der Körperflüssigkeit in der angepressten Körperpartie (66) aufweist.

27. Analysegerät nach Anspruch 26, **dadurch gekennzeichnet, dass** das Kompressionsorgan durch einen vorzugsweise konischen Pressring (64) zur Auswölbung der Körperpartie (66) gegen das Stechorgan (18) gebildet ist.

28. Analysegerät nach einem der Ansprüche 1 bis 27, **dadurch gekennzeichnet, dass** die Stecheinheit (16) und/oder die Testbandeinheit (20) eine zu der Aufnahme (12) hin verjüngte distale Kopfpartie aufweisen.

29. Analysegerät nach einem der Ansprüche 1 bis 28, **dadurch gekennzeichnet, dass** ein im Bereich der Aufnahme gespannter Abschnitt des Testbands (22) als Widerlager (14) eine Referenzposition für den Stechhub bildet.

30. Analysegerät nach einem der Ansprüche 1 bis 29, **dadurch gekennzeichnet, dass** das Widerlager (14) durch eine Verschiebe- oder Kompressionsbewegung der Aufnahme (12) in die Freigabe und Wirkstellung bringbar ist.

31. Analysegerät nach einem der Ansprüche 1 bis 30, **dadurch gekennzeichnet, dass** die Detektionseinheit (24) auf mehrere auf einem Abschnitt des Testbands (22) nebeneinander liegende Messpunkte (128) zur Dosierungskontrolle der applizierten Körperflüssigkeit ausgerichtet ist

32. Analysegerät nach einem der Ansprüche 1 bis 31, **gekennzeichnet durch** eine Bandumlenkeinheit (134,136) zum Ausziehen einer Schlaufe (140) des Testbandes (22) in den Bereich der Aufnahme (12).

33. Analysegerät nach Anspruch 32, **dadurch gekennzeichnet, dass** die Bandumlenkeinheit (134,136) eine das Testband führende, hin und her bewegliche Umlenkrolle (134) aufweist.

34. Analysegerät nach Anspruch 32, **dadurch gekennzeichnet, dass** die Bandumlenkeinheit (134,136) eine das Testband führende federelastische Umlenkzunge aufweist.

35. Analysegerät nach einem der Ansprüche 32 bis 34, **dadurch gekennzeichnet, dass** die Bandumlenkeinheit (134,136) einen Antrieb (136), vorzugsweise einen Schwenkhebelantrieb für eine hin und her gehende Bewegung zum Ausziehen und Rückholen der Bandschlaufe (140) aufweist.

36. Analysegerät nach einem der Ansprüche 1 bis 35, **gekennzeichnet durch** mehrere in dem Gehäuse (10) angeordnete Gerätekomponenten (16,20,24), zumindest umfassend eine Stecheinheit (16) zum Einstechen eines Stechorgans (18) in die Körperpartie (66), eine Testmitteleinheit (20') zur Applikation von aus der Körperpartie (66) austretender Körperflüssigkeit auf ein Testmittel, insbesondere Testband (22) oder Teststreifen, und gegebenenfalls eine Detektionseinhelt (24) zur Untersuchung der auf dem Testmittel (22) applizierten Körperflüssigkeit, wobei mindestens zwei der Gerätekomponenten (16,20,24) wechselweise in ihre jeweilige Funktionsstellung bezüglich der Aufnahme (12) bewegbar sind, wobei die Gerätekomponenten (16,20,24) auf einer gemeinsamen Tragstruktur (150) angeordnet sind, und wobei die Tragstruktur (150) zur Positionierung der Gerätekomponenten (16,20,24) in ihrer jeweiligen Funktionsstellung gegenüber dem Gehäuse (10) bewegbar ist.

37. Analysegerät nach Anspruch 36, **dadurch gekennzeichnet, dass** die Tragstruktur (150) mittels einer Positioniervorrichtung (158) vorzugsweise auf einer bogenförmigen Bahnkurve in die jeweilige Zielposition der Gerätekomponenten (16,20,24) bewegbar ist.

38. Analysegerät nach Anspruch 36 oder 37, **dadurch gekennzeichnet, dass** die Tragstruktur (150) durch eine mit Befestigungselementen (152,154,156) für die Gerätekomponenten (16,20,24) versehene Plattform gebildet ist.

39. Analysegerät nach einem der Ansprüche 36 bis 38, **dadurch gekennzeichnet, dass** die Tragstruktur (150) Klemm- , Schraub- oder Rastorgane zur vorzugsweise lösbaren Fixierung der Gerätekomponenten (16,20,24) aufweist.

40. Analysegerät nach einem der Ansprüche 36 bis 39, **dadurch gekennzeichnet, dass** die Tragstruktur (150) raster- oder gitterförmig angeordnete Befestigungspunkte für die Gerätekomponenten (16,20,24) aufweist.

41. Analysegerät nach einem der Ansprüche 36 bis 40, **dadurch gekennzeichnet dass** die Testmitteleinheit ein Magazin (166) insbesondere ein Trommelmagazin oder Stapelmagazin zur Verarbeitung einer Mehrzahl von Teststreifen aufweist.

## Claims

1. Analytical instrument for body fluids and in particular a portable blood sugar measuring instrument comprising
a) a housing (10) provided with a receiving element (12) for engaging a body part (66), preferably a fingertip of a test person,
b) a lancing unit (16) having a lancing element (18) that can pierce the body part (66),
c) a test tape unit (20) having a test tape (22) for applying body fluid issuing from the body part (66), and
d) a detecting unit (24) for examining the body fluid applied to a section of the test tape (22)
e) an abutment (14) for the body part (66) which can be brought into a release position and an operating position relative to the receiving element (12),
**characterized in that**
f) the abutment (14) is provided with a piercing opening (32) for the lancing element (18), wherein the lancing member (18) can be lanced in a linear lancing stroke into the body part (66) resting against the abutment (14) and the abutment in the operating position when the body part rests against it creates a reference position for a defined lancing.

2. Analytical instrument according to claim 1, **characterized in that** the section of test tape to be loaded with body fluid can be transported into the area of the receiving element (12) in the release position of the abutment (14).

3. Analytical instrument according to claim 1 or 2, **characterized in that** the lancing unit (16) and the test tape unit (20) can be alternately brought into an functional position relative to the receiving element (12) by means of a positioning device (70).

4. Analytical instrument according to any one of the claims 1 to 3, **characterized in that** the abutment (14) is formed by a front surface (26) of the lancing unit (16) which faces the receiving element (12) in the operating position.

5. Analytical instrument according to any one of the claims 1 to 4, **characterized in that** the lancing unit (16) and the test tape unit (20) can be moved as a common assembly relative to the housing (10) in a defined positional relation relative to one another.

6. Analytical instrument according to any one of the claims 1 to 5, **characterized in that** the assembly consisting of lancing unit (16) and test tape unit (20) can be moved linearly in a linear guide (74).

7. Analytical instrument according to any one of the claims 1 to 5, **characterized in that** the assembly consisting of lancing unit (16) and test tape unit (20) can be moved on a curved path which is in particular U-shaped.

8. Analytical instrument according to any one of the claims 1 to 7, **characterized in that** a linkage (76, 78) is arranged between the housing (10) and a common support structure (72) for the lancing unit (16) and test tape unit (20).

9. Analytical instrument according to any one of the claims 1 to 4, **characterized in that** the lancing unit (16) and test tape unit (20) can be moved alternately into the area of the receiving element (12) on separate paths by means of specially dedicated delivery means (80) that are preferably coupled to one another.

10. Analytical instrument according to claim 9, **characterized in that** the delivery means (80) comprise a rack-and-pinion drive (86, 88) or rotary lever drive.

11. Analytical instrument according to any one of the claims 1 to 10, **characterized in that** the abutment (14) is formed by a movable tape guide (90) for the test tape (22).

12. Analytical instrument according to any one of the claims 1 to 11, **characterized in that** the abutment (14) can advantageously be moved to a limited extent in the lancing direction by means of a curve control device (96) and in particular a switchable cam disk.

13. Analytical instrument according to claim 11 or 12, **characterized in that** the lancing unit (16) and test tape unit (20) are arranged in fixed positions in the instrument.

14. Analytical instrument according to any one of the claims 11 to 13, **characterized in that** the tape guide (90) distally spans the lancing unit (16) preferably in an arched manner towards the receiving element (12).

15. Analytical instrument according to any one of the claims 1 to 14, **characterized in that** the test tape unit (20) has a reciprocating deflecting head (56) to form a tape loop in the area of the receiving element (12).

16. Analytical instrument according to claim 15, **characterized in that** the deflecting head (56) is tapered in a convex or angled manner towards the receiving element (12).

17. Analytical instrument according to claim 15 or 16, **characterized in that** the detection unit (24) which preferably employs reflection photometry is located in the deflecting head (56).

18. Analytical instrument according to any one of the claims 1 to 17, **characterized in that** the abutment (14) in the operating position forms a reference means for determining the magnitude of the lancing stroke.

19. Analytical instrument according to any one of the claims 1 to 18, **characterized in that** the test tape (22) is provided with a piercing opening (32) for the lancing element (18).

20. Analytical instrument according to any one of the claims 1 to 19, **characterized in that** the lancing unit (16) has an adjusting device (38) which can preferably be operated from outside the housing for adjusting the lancing stroke relative to the abutment (14).

21. Analytical instrument according to any one of the claims 1 to 20, **characterized in that** the lancing unit (16) has a trigger (40) preferably arranged on the outside of the housing for manually actuating the lancing stroke.

22. Analytical instrument according to any one of the claims 1 to 21, **characterized in that** the test tape unit (20) comprises a tape cassette (42) for transporting the test tape in sections preferably by means of winding spools (44, 48).

23. Analytical instrument according to any one of the claims 1 to 22, **characterized in that** a plurality of lancets are stored as a lancing element (18) in a preferably exchangeable lancet magazine (36) of the lancing unit (16).

24. Analytical instrument according to any one of the claims 1 to 23, **characterized in that** the test tape (22) has more than 15 and preferably more than 50 sections of test tape (50) that can be positioned relative to the receiving element (12) by advancing the tape for successive examinations of body fluid.

25. Analytical instrument according to claim 24, **characterized in that** the ratio of stored lancing elements to test tape sections is between 1:1 and 1:50

26. Analytical instrument according to any one of the claims 1 to 25, **characterized in that** the receiving element (12) has a compression element (64) for increasing the internal pressure of the body fluid in the pressed body part (66).

27. Analytical instrument according to claim 26, **characterized in that** the compression element is formed by a preferably conical press ring (64) for bulging the body part (66) against the lancing element (18).

28. Analytical instrument according to any one of the claims 1 to 28, **characterized in that** the lancing unit (16) and/or the test tape unit (20) have a distal head member which tapers towards the receiving element (12).

29. Analytical instrument according to any one of the claims 1 to 28, **characterized in that** a section of the test tape (22) that is clamped in the area of the receiving element acts as an abutment (14) to form a reference position for the lancing stroke.

30. Analytical instrument according to any one of the claims 1 to 29, **characterized in that** the abutment (14) can be moved into the release and operating position by a displacement or compression movement of the receiving element (12).

31. Analytical instrument according to any one of the claims 1 to 30, **characterized in that** the detection unit (24) is aligned with several measuring points (128) located next to one another on one section of the test tape (22) in order to check the dose of the applied body fluid.

32. Analytical instrument according to any one of the claims 1 to 31, **characterized by** a tape deflection unit (134, 136) for pulling out a loop (140) of the test tape (22) in the area of the receiving element (12).

33. Analytical instrument according to claim 32, **characterized in that** the tape deflection unit (134, 136) has a reciprocating deflecting roller (134) which guides the test tape.

34. Analytical instrument according to claim 32, **characterized in that** the tape deflection unit (134, 136) has a spring elastic deflection tongue which guides the test tape.

35. Analytical instrument according to any one of the claims 32 to 24, **characterized in that** the tape deflection unit (134, 136) has a drive (136) and preferably a pivoting lever drive for a backwards and forwards movement to pull out and retract the tape loop (140).

36. Analytical instrument according to any one of the claims 1 to 35, **characterized by** several instrument components (16, 20, 24) arranged in the housing (10) and at least comprising a lancing unit (16) to lance a lancing element (18) into the body part (66), a test means unit (20') for applying body fluid discharged from the body part (66) onto a test means, in particular a test tape (22) or test strip, and optionally a detection unit (24) for examining the body fluid applied to the test means (22), wherein at least two of the instrument components (16, 20, 24) can be moved alternately into their respective operating position relative to the receiving element (12), wherein the instrument components (16, 20, 24) are arranged on a common support structure (150) and wherein the support structure (150) can be moved relative to the housing (10) in order to position the instrument components (16, 20, 24) in their respective operating position.

37. Analytical instrument according to claim 36, **characterized in that** the support structure (150) can be moved by means of a positioning device (158) preferably on an arc-shaped path into the respective end position of the instrument components (16, 20, 24).

38. Analytical instrument according to claim 36 or 37, **characterized in that** the support structure (150) is formed by a platform provided with attachment elements (152, 154, 156) for the instrument components (16, 20, 24).

39. Analytical instrument according to any one of the claims 36 to 38, **characterized in that** the support structure (150) has clamping, screwing or locking elements for the preferably detachable attachment of the instrument components (16, 20, 24).

40. Analytical instrument according to any one of the claims 36 to 39, **characterized in that** the support structure (150) has attachment points for the instrument components (16, 20, 24) arranged in a raster or grid pattern.

41. Analytical instrument according to any one of the claims 36 to 40, **characterized in that** the test means unit has a magazine (166) in particular a drum magazine or stack magazine to process a plurality of test strips.

## Revendications

1. Appareil d'analyse de liquides organiques, en particulier lecteur de glycémie portatif, comprenant :
a) un boîtier (10) pourvu d'un élément de réception (12) pour l'engagement d'une partie du corps (66), de préférence de la pulpe du doigt d'un sujet,
b) une unité de piqûre (16) comportant un organe de piqûre (18) permettant de piquer ladite partie du corps (66),
c) une unité de bande de test (20) comportant une bande de test (22) pour l'application d'un liquide organique sortant de ladite partie du corps (66),
d) une unité de détection (24) pour analyser le liquide organique appliqué sur une portion de la bande de test (22),
e) une butée (14) pour ladite partie du corps (66), qui peut être amenée dans une position inactive et une position active par rapport à l'élément de réception (12),
**caractérisé en ce que**
f) la butée (14) est pourvue d'un orifice traversant (32) pour l'organe de piqûre (18), ledit organe de piqûre (18) pouvant piquer ladite partie du corps (66) appliquée contre la butée (14) selon un mouvement de piqûre linéaire et la butée (14) créant dans la position active, lorsque ladite partie du corps est appliquée, une position de référence pour une piqûre définie.

2. Appareil d'analyse selon la revendication 1, **caractérisé en ce que** la portion à alimenter en liquide organique de la bande de test peut être transportée dans la position inactive de la butée (14) dans la région de l'élément de réception (12).

3. Appareil d'analyse selon la revendication 1 ou 2, **caractérisé en ce que** l'unité de piqûre (16) et l'unité de bande de test (20) peuvent être amenées au moyen d'un dispositif de positionnement (70) à tour de rôle dans une position fonctionnelle par rapport à l'élément de réception (12).

4. Appareil d'analyse selon l'une des revendications 1 à 3, **caractérisé en ce que** la butée (14) est formée par une face frontale (26), tournée vers l'élément de réception (12) dans la position active, de l'unité de piqûre (16).

5. Appareil d'analyse selon l'une des revendications 1 à 4, **caractérisé en ce que** l'unité de piqûre (16) et l'unité de bande de test (20) forment, dans une relation de position réciproque définie, un bloc commun déplaçable par rapport au boîtier (10).

6. Appareil d'analyse selon l'une des revendications 1 à 5, **caractérisé en ce que** ledit bloc formé de l'unité de piqûre (16) et de l'unité de bande de test (20) est translatable en ligne droite dans un guide linéaire (74).

7. Appareil d'analyse selon l'une des revendications 1 à 5, **caractérisé en ce que** ledit bloc formé de l'unité de piqûre (16) et de l'unité de bande de test (20) est déplaçable sur une trajectoire courbe, notamment en forme de U.

8. Appareil d'analyse selon l'une des revendications 1 à 7, **caractérisé en ce qu**'un mécanisme à bielle (76, 78) est disposé entre le boîtier (10) et une structure de support commune (72) pour l'unité de piqûre (16) et l'unité de bande de test (20).

9. Appareil d'analyse selon l'une des revendications 1 à 4, **caractérisé en ce que** l'unité de piqûre (16) et l'unité de bande de test (20) peuvent être déplacées par des moyens d'avance (80) dédiés et de préférence couplés entre eux, sur des trajectoires séparées, à tour de rôle dans la région de l'élément de réception (12).

10. Appareil d'analyse selon la revendication 9, **caractérisé en ce que** les moyens d'avance (80) sont formés par un entraînement à crémaillère (86, 88) ou un entraînement à levier pivotant.

11. Appareil d'analyse selon l'une des revendications 1 à 10, **caractérisé en ce que** la butée (14) est formée par un guide de bande mobile (90) pour la bande de test (22).

12. Appareil d'analyse selon l'une des revendications 1 à 11, **caractérisé en ce que** la butée (14) est déplaçable de façon limitée dans le sens de la piqûre par une commande à came (96), en particulier un disque à came commutable.

13. Appareil d'analyse selon la revendication 11 ou 12, **caractérisé en ce que** l'unité de piqûre (16) et l'unité de bande de test (20) sont disposés de manière fixe dans l'appareil.

14. Appareil d'analyse selon l'une des revendications 11 à 13, **caractérisé en ce que** le guide de bande (90) enjambe l'unité de piqûre (16) distalement, de préférence en forme d'arc, en direction de l'élément de réception (12).

15. Appareil d'analyse selon l'une des revendications 1 à 14, **caractérisé en ce que** l'unité de bande de test (20) comporte une tête de renvoi (56) à mouvement de va-et-vient, servant à former une boucle de bande positionnable dans la région de l'élément de réception (12).

16. Appareil d'analyse selon la revendication 15, **caractérisé en ce que** ladite tête de renvoi (56) est amincie de façon convexe ou angulaire, en direction de l'élément de réception (12).

17. Appareil d'analyse selon la revendication 15 ou 16, **caractérisé en ce que** l'unité de détection (24) fonctionnant de préférence par photométrie à réflexion, est montée dans la tête de renvoi (56).

18. Appareil d'analyse selon l'une des revendications 1 à 17, **caractérisé en ce que** la butée (14) forme dans la position active un moyen de référence pour la définition de la hauteur du mouvement de piqûre.

19. Appareil d'analyse selon l'une des revendications 1 à 18, **caractérisé en ce que la** bande de test (22) est pourvue d'un orifice traversant (32) pour l'organe de piqûre (18).

20. Appareil d'analyse selon l'une des revendications 1 à 19, **caractérisé en ce que** l'unité de piqûre (16) comporte un moyen d'ajustage (38), de préférence actionnable depuis le côté extérieur du boîtier, pour le réglage du mouvement de piqûre par rapport à la butée (14).

21. Appareil d'analyse selon l'une des revendications 1 à 20, **caractérisé en ce que** l'unité de piqûre (16) comporte un déclencheur (40), de préférence disposé sur le côté extérieur du boîtier, pour le déclenchement manuel du mouvement de piqûre.

22. Appareil d'analyse selon l'une des revendications 1 à 21, **caractérisé en ce que** l'unité de bande de test (20) est formée par une cassette de bande (42) pour le transport par portions de la bande de test, de préférence au moyen de bobines d'enroulement (44, 48).

23. Appareil d'analyse selon l'une des revendications 1 à 22, **caractérisé en ce qu**'une pluralité de lancettes comme organe de piqûre (18) sont stockées dans un magasin à lancettes (36), de préférence échangeable, de l'unité de piqûre (16).

24. Appareil d'analyse selon l'une des revendications 1 à 23, **caractérisé en ce que** la bande de test (22) comporte plus de 15, de préférence plus de 50 portions de bande de test positionnables par rapport à l'élément de réception (12) par avance de la bande, pour des analyses successives du liquide organique.

25. Appareil d'analyse selon la revendication 24, **caractérisé en ce que** le rapport entre organes de piqûre et portions de bande de test stockés est compris entre 1:1 et 1:50.

26. Appareil d'analyse selon l'une des revendications 1 à 25, **caractérisé en ce que** l'élément de réception (12) comporte un organe de compression (64) servant à augmenter la pression interne du liquide organique dans ladite partie du corps (66) appliquée.

27. Appareil d'analyse selon la revendication 26, **caractérisé en ce que** ledit organe de compression est formé par une bague de compression (64) de préférence conique, servant à faire saillir la partie du corps (66) contre l'organe de piqûre (18).

28. Appareil d'analyse selon l'une des revendications 1 à 27, **caractérisé en ce que** l'unité de piqûre (16) et/ou l'unité de bande de test (20) comportent une partie de tête distale amincie vers l'élément de réception (12).

29. Appareil d'analyse selon l'une des revendications 1 à 28, **caractérisé en ce qu**'une portion tendue au niveau dudit élément de réception de la bande de test (22) forme en tant que butée (14) une position de référence pour le mouvement de piqûre.

30. Appareil d'analyse selon l'une des revendications 1 à 29, **caractérisé en ce que** la butée (14) peut être amenée dans les positions inactive et active par un mouvement de translation ou de compression de l'élément de réception (12).

31. Appareil d'analyse selon l'une des revendications 1 à 30, **caractérisé en ce que** l'unité de détection (24) est orientée sur plusieurs points de mesure (128) disposés côte à côte sur une portion de la bande de test (22), pour contrôler le dosage du liquide organique appliqué.

32. Appareil d'analyse selon l'une des revendications 1 à 31, **caractérisé par** une unité de renvoi de bande (134, 136) servant à déployer une boucle (140) de la bande de test (22) dans la région de l'élément de réception (12).

33. Appareil d'analyse selon la revendication 32, **caractérisé en ce que** l'unité de renvoi de bande (134, 136) comporte un rouleau de renvoi (134) à mouvement de va-et-vient, qui guide ladite bande de test.

34. Appareil d'analyse selon la revendication 32, **caractérisé en ce que** l'unité de renvoi de bande (134, 136) comporte une lame de renvoi à élasticité de ressort, qui guide ladite bande de test.

35. Appareil d'analyse selon l'une des revendications 32 à 34, **caractérisé en ce que** l'unité de renvoi de bande (134, 136) comporte un entraînement (136), de préférence un entraînement à levier pivotant pour un mouvement de va-et-vient servant à déployer et à rappeler la boucle de bande (140).

36. Appareil d'analyse selon l'une des revendications 1 à 35, **caractérisé par** plusieurs composants d'appareil (16, 20, 24) montés dans le boîtier (10), comprenant au moins une unité de piqûre (16) servant à faire pénétrer un organe de piqûre (18) dans ladite partie du corps (66), une unité de moyen de test (20') servant à appliquer du liquide organique sortant de ladite partie du corps (66) sur un moyen de test, en particulier la bande de test (22) ou bandelette de test, et le cas échéant une unité de détection (25) servant à analyser le liquide organique appliqué sur le moyen de test (22), au moins deux desdits composants d'appareil (16, 20, 24) pouvant être amenées à tour de rôle dans leur position fonctionnelle respective par rapport à l'élément de réception (12), lesdits composants d'appareil (16, 20, 24) étant montés sur une structure de support (150) commune, et ladite structure de support (150) étant déplaçable pour permettre le positionnement des composants d'appareil (16, 20, 24) dans leur position fonctionnelle respective par rapport au boîtier (10).

37. Appareil d'analyse selon la revendication 36, **caractérisé en ce que** la structure de support (150) peut être déplacée au moyen d'un dispositif de positionnement (158), de préférence sur une trajectoire courbe, dans la position cible respective des composants d'appareil (16, 20, 24).

38. Appareil d'analyse selon la revendication 36 ou 37, **caractérisé en ce que** la structure de support (150) est formée par une plateforme pourvue d'éléments de fixation (152, 154, 156) pour les composants d'appareil (16, 20, 24).

39. Appareil d'analyse selon l'une des revendications 36 à 38, **caractérisé en ce que** la structure de support (150) comporte des organes à serrer, à visser ou à encliqueter, servant à la fixation de préférence amovible des composants d'appareil (16, 20, 24).

40. Appareil d'analyse selon l'une des revendications 36 à 39, **caractérisé en ce que** la structure de support (150) comporte des points de fixation disposés en forme de trame ou de grille pour les composants d'appareil (16, 20, 24).

41. Appareil d'analyse selon l'une des revendications 36 à 40, **caractérisé en ce que** l'unité de moyen de test comporte un magasin (166), en particulier un magasin à tambour ou un magasin à pile pour la mise en oeuvre d'une pluralité de bandelettes de test.
